(19) Europäisches Patentamt
European Patent Office
Office européen des brevets

(11) Publication number: **0 092 350**
**B1**

(12) # EUROPEAN PATENT SPECIFICATION

(45) Date of publication of the patent specification: **11.09.85**

(21) Application number: **83301982.1**

(22) Date of filing: **08.04.83**

(51) Int. Cl.[4]: **C 07 C 51/353,** C 07 C 67/347, C 07 C 53/122, C 07 C 53/124, C 07 C 53/126, C 07 C 69/24, B 01 J 23/46, B 01 J 27/08

(54) **A process for converting olefins to carboxylic acids or esters using soluble iridium compounds.**

(30) Priority: **12.04.82 US 367786**

(43) Date of publication of application: **26.10.83 Bulletin 83/43**

(45) Publication of the grant of the patent: **11.09.85 Bulletin 85/37**

(84) Designated Contracting States: **DE FR GB NL**

(56) References cited:
**DE - A - 2 364 446**
**FR - A - 1 484 367**
**FR - A - 2 147 185**
**US - A - 2 013 338**
**US - A - 3 944 603**

(73) Proprietor: **Exxon Research and Engineering Company, P.O.Box 390 180 Park Avenue, Florham Park New Jersey 07932 (US)**

(72) Inventor: **Pruett, Roy Lavelle, 21 Wellings Road, New Providence New Jersey (US)**
Inventor: **Burk, Patrick Lee, 111 Lincoln Drive West, Ambler Pennsylvania (US)**

(74) Representative: **Field, Roger Norton et al, ESSO Engineering (Europe) Ltd. Patents & Licences Apex Tower High Street, New Malden Surrey KT3 4DJ (GB)**

**EP 0 092 350 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

BUNDESDRUCKEREI BERLIN

## Description

This invention relates to a homogeneous process for converting olefins to carboxylic acids and/or esters. More particulary, olefins are contacted with a formate ester or trans-esterification product thereof in the presence of a soluble iridium compound and iodide promoter in a carboxylic acid solvent.

It is known that olefins can be converted to carboxylic acids or esters in the presence of a hydroxy compound, carbon monoxide or carbon dioxide and Group VIII catalyst. U. S. Patent 2 739 169 discloses the reaction of CO and steam with organic compounds such as olefins, alcohols, ethers and esters to produce carboxylic acids and/or anhydrides. Metal carbonyls are stated to be catalysts with Ni, Co, Fe, Cr, Mo, Ru, Pd, Pt, Re, Os and Ir being described as typical carbonylforming materials. If steam is replaced by carboxylic acid, an anhydride is produced in the place of a carboxylic acid.

U. S. Patent 3 816 489 teaches a process for preparing carboxylic acids from olefins, CO and $H_2O$ using an iridium catalyst plus an iodide promoter wherein the iodine to iridium ratio is maintained between critical limits. U. S. Patent 4 000 170 describes a similar reaction with the iridium in the catalyst system being at least 50 wt.% in a highly catalytically active form as defined by a characteristic infrared spectrum. U. S. Patent 4 132 734 is directed to the addition of cobalt, iron or manganese to a catalyst system containing a rhodium or iridium compound and a halogen to produce carboxylic acids from olefins, CO and $H_2O$. I. S. Kolomnikov et al., in Kinetics and Catalysis, 13, 227 (1972) state that group VIII metals catalyse hydrogenation of olefins in the presence of formic acid, and if a triphenylphosphine complex of said metals is used, the addition of formic acid to olefin to form a carboxylic acid may also occur. $(Ph_3P)_3RhCl$ is stated to convert cyclohexene and HCOOH to cyclohexane carboxylic acid in 10% yield. Reaction conditions are not stated. T. Mizorogi, Shokubai (Catalysts), 19, 90—95 (1977) reviews the synthesis of $C_2$-containing oxygen compounds using CO. The author cites a reference 10 indicating that $IrCl_4 \cdot H_2O-CH_3I$ catalyst shows isomerization activity. This reference, however, is to unpublished data.

U. S. Patent No. 3 839 428 which was reissued as Re. 29,981 teaches a process for converting formic acid esters to the corresponding carboxylic acids which comprises contacting the ester with carbon monoxide (CO) at an elevated CO pressure. Although the reaction will occur in the absence of a catalyst, Group VIII metals such as iron, cobalt and nickel, Group IIb metals and halogens may be advantageously employed to increase the conversion rate. U. S. Patent 3 488 383 concerns the selective decomposition of formic acid in admixture with another aliphatic acid or ester of an aliphatic acid, e. g., methyl formate. The process comprises contacting the formic acid-containing mixture with a soluble complex compound of a Group VIII metal or rhenium. The complex compound is a compound of Pt, Os, Rh, or preferably Ru or Ir. Formate esters are unreactive and may be present as part ot the solvent system. Example 1 indicates that at least part of the formic acid is decomposed to $H_2$ und $CO_2$.

U. S. 2 013 338 discloses a process for producing an aliphatic carboxylic acid by reacting an olefinic hydrocarbon with formic acid under a pressure of 25—900 atmospheres in the presence of certain catalysts, e. g. calcium iodide supported on activated charcoal.

It has been discovered that olefins can be converted to carboxylic acids or carboxylic acid esters without the presence of carbon monoxide or hydroxyl containing compound. The present homogeneous process for preparing a carboxylic acid ester comprises contacting an olefin and a formate ester with a catalytically effective amount of a soluble iridium salt or compound and an iodide promoter in the presence of a carboxylic acid at a temperature from 150 to 300° C.

In another embodiment a homogeneous process for preparing a carboxylic acid comprises contacting an olefin and formic acid with a catalytically effective amount of a soluble iridium salt or compound and an iodide promoter at a temperature of from 150 to 300° C.

As noted above, the process of the invention does not require CO or a hydroxy containing compound such as water or alcohol. Moreover, no special high pressure apparatus is required since autogenous pressures may be employed.

Carboxylic acids and esters thereof are produced as illustrated by the following overall reactions employing a terminal olefin as reactant:

$$HCOOR^1 + R^2CH{=}CH_2 \xrightleftharpoons[RCOOH]{Ir\ Cat.} R^2CH_2CH_2COOR^1 + R^2\underset{\underset{\displaystyle CH_3}{|}}{C}HCOOR^1 \quad (I)$$

$$HCOOH + R^2CH{=}CH_2 \xrightleftharpoons[HCOOH/RCOOH]{Ir\ Cat.} R^2CH_2CH_2COOH + R^2\underset{\underset{\displaystyle CH_3}{|}}{C}HCOOH \quad (II)$$

By suitable adjustments of reaction components either carboxylic acids or esters may be selectively obtained as the final product. As illustrated above, a mixture of acids and/or esters is typically produced.

Formate esters may have the formula $HCOOR^1$ where $R^1$ is aliphatic, cycloaliphatic, araliphatic or aryl, preferably $C_1-C_{20}$ aliphatic, $C_3-C_{12}$ cycloaliphatic, $C_7-C_{16}$ araliphatic or $C_6-C_{12}$ aryl, more preferably $C_1-C_8$ aliphatic, $C_4-C_8$ cycloaliphatic, $C_7-C_{12}$ araliphatic and most preferably $C_1-C_6$ alkyl, $C_5-C_6$ cycloalkyl, $C_7-C_{10}$ aralkyl and especially methyl, ethyl, propyl or butyl. The $R^1$ radical may also bear unreactive substituents such as halo, cyano and the like. Examples of suitable formate esters include methyl formate, ethyl formate, propyl formate, butyl formate, isobutyl formate, cyclohexyl formate, phenyl formate, benzyl formate, phenethyl formate, octyl formate and dodecyl formate.

The olefin reactants may be linear, branched or cyclic monoolefins, diolefins or polyolefins, and preferably have the formula $R^2R^3C = CR^4R^5$ where $R^2$, $R^3$, $R^4$ and $R^5$ are independently hydrogen, $C_1-C_{12}$ aliphate, $C_3-C_{10}$ cycloaliphatic, $C_7-C_{14}$ araliphatic or $C_6-C_{12}$ aryl, preferably $C_1-C_8$ aliphatic, $C_5-C_8$ cycloaliphatic, $C_7-C_{12}$ araliphatic or $C_6-C_{10}$ aryl, with provisos that at least two of $R^2$ to $R^5$ are hydrogen and any two of $R^2$ to $R^5$ may be joined together to form a $C_3$ to $C_{10}$ ring. Highly branched olefins generally exhibit very low reactivities or are unreactive and are therefore not desirable as reactants. The aliphatic radicals may be saturated hydrocarbyl radicals giving rise to monoolefin reactants, or may be ethylenically unsaturated hydrocarbyl radicals giving diolefins or polyolefins as reactants. In homogeneous catalytic reactions of the type set forth in the present processes, the olefin typically reacts with metal to form a metal-olefin intermediate complex. Thus any olefin capable of complexing with the catalyst may be employed.

Example of olefin reactants are ethylene, propylene, butene-1, butene-2, butadiene, isoprene, pentene-1, 1,4-pentadiene, 4,4-dimethylpentene-1, 2,3-dimethylhexene-1, 3-ethylhexene-1, hexene-2, cyclohexene, styrene, allyl benzene, stilbene, dicyclopentadiene, vinyl cyclohexene, octene-1, 1,7-octadiene, octene-4, dodecene-1, octadecene-1, fulvene, indene, eicosene-1, bicyclo[2.2.1]hepta-2,5-diene, 1,4-cyclooctadiene and limonene. Linear and cyclic monoolefins and diolefins are preferred with terminal monoolefins and diolefins being especially preferred.

The processes of the invention are homogeneous and are carried out using carboxylic acid solvents or mixtures thereof with inert organic solvents. In the case of reaction (II) above, the solvent may be either formatic acid, another carboxylic acid or mixtures with inert organic solvent. Mixture of carboxylic acids as solvents may also be used in either reactions (I) or (II) above, e. g., a mixture of formic and acetic acids. Suitable carboxylic acids are of the formula RCOOH where R is alipahtic, cycloaliphatic, araliphatic or aromatic. Preferred carboxylic acids are those wherein R is $C_1-C_{20}$ aliphatic, more preferably $C_1-C_{10}$ alkyl and most preferably $C_1-C_{16}$ alkyl. Formic, acetic, propionic and butyric acids are especially preferred. Other inert organic solvents such as ethers, ketones, amides, sulfoxides and hydrocarbons may be present provided that the carboxylic acid comprises at least about 50 wt.% of the solvent mixture. Lower amounts of carboxylic acid in the solvent system may lead to decreased reactivity. A small amount of water, i. e., less than about 10 wt.%, may be present. Larger amounts of water lead to decreased yields. It is preferred to have less than about 5 wt.% of water present in the solvent system.

While not wishing to be bound to any particular theory or reaction mechanism, the presence of carboxylic acid is essential in the formation of esters according to reaction (I) above. One explanation for this requirement is summarised by the following reactions:

$$HCOOR^1 + RCOOH \rightleftharpoons HCOOH + RCOOR^1 \qquad (A)$$

$$HCOOH + R^2CH{=}CH_2 \xrightleftharpoons{\text{Ir Cat.}} R^2CH_2CH_2COOH + R^2{-}\underset{\displaystyle CH_3}{\underset{|}{C}H}{-}COOH \qquad (B)$$

$$\begin{matrix} R^2CH_2CH_2COOH \\ R^2\underset{\displaystyle CH_3}{\underset{|}{C}H}COOH \end{matrix} \xrightleftharpoons{RCOOR^1} \begin{matrix} R^2CH_2CH_2COOR^1 \\ R^2{-}\underset{\displaystyle CH_3}{\underset{|}{C}H}COOR^1 \end{matrix} + RCOOH \qquad (C)$$

Reactions (A) and (C) are transesterification reactions which consume and regenerate RCOOH, respectively. Reaction (B) corresponds to reaction (II) above and provides an explanation for this particular embodiment. If carboxylic acids are desired as products of olefin reaction, i. e., reaction (II) above, then formic acid may be both reactant and solvent. Other carboxylic acids may, however, be present as solvent.

Suitable catalysts or catalyst precursors for the conversion of olefins to carboxylic acid and/or esters are iridium salts or compounds which are soluble in the reaction medium chosen. Iridium salts or compounds are simple salts or compounds such as halides or oxides, and complexes containing halides, phosphines, amines including heterocyclic amines, arsines, cyanides, sulfides, esters, betadiketonates, carboxylates, hydrido, sulfites, nitro and the like as ligands. The complexes may also be organometallic iridium complexes containing both $\sigma$ and $\pi$ bonded organic ligands, e. g., carbonyl,

alkyl, alkenes and alkynes. Exemples of iridium salts and complexes are
[Ir(cyclooctadiene)Cl]$_2$, $Na_2IrCl_6 \cdot x\,H_2O$, $H_2IrCl_6 \cdot x\,H_2O$, $HIrCO[P(C_6H_5)_3]_3$, $ClIrCO(PEt_3)_2$, $IrCl_3 \cdot x\,H_2O$, $IrI_3 \cdot x\,H_2O$, $Na_3Ir(NO_2)_6 \cdot x\,H_2O$, $K_3Ir(CN)_6$, $[(C_5H_5)_2Ir]NO_3$, $Ir_4(CO)_{12}$, $IrH_3[P(C_6H_5)_3]_3$, 1,2,3-py$_3$IrCl$_3$, trans-[Irpy$_4$Cl$_2$]Cl and $(C_8H_{12})_2\,IrSnCl_3$.

Preferred salts are
[Ir(cyclooctadiene)Cl]$_2$, $IrCl_3 \cdot x\,H_2O$, $IrBr_3 \cdot x\,H_2O$ and $IrI_3 \cdot x\,H_2O$.

[Ir(cyclooctadiene)Cl]$_2$, $IrCl_3 \cdot x\,H_2O$ and $IrI_3 \cdot x\,H_2O$ are especially preferred. The symbol x indicates differing degrees of hydration and varies from 0 to 12.

Iodides generally are promoters or co-catlysts for the conversion of olefins to carboxylic acids and/or esters. Examples of suitable promoters include iodine, HI, organic iodides, and ammonium or phosphonium iodides of the formulae $N(R^6)_4I$ and $P(R^6)_4I$ where each $R^6$ is independently hydrogen or $C_1-C_6$ alkyl. Preferred iodides are alkyl iodides of the formula $R^7I$ where $R^7$ is $C_1-C_{10}$ alkyl.

Catalyst concentrations may range from about 0.001 to 15 wt.% or iridium calculated as metal, based on the reaction mixture, preferably from 0.01 to 10 wt.%, especially 0.01 to 2 wt.%. The amount of iodide promoter is not critical, but high iodide: Ir ratios may retard the reaction. Amounts in excess of about 0.01 wt.%, based on the reaction mixture may be employed, preferably from about 0.05 to 2 wt.%. It is most preferred to maintain the I : Ir ratio at about 10 : 1.

In addition to making carboxylic acid esters, the catalyst system of the invention may also be employed to convert olefins to carboxylic acids. In this embodiment, an olefin as defined above is contacted with formic acid in the presence of catalyst to produce a carboxylic acid. An example of this reaction is the conversion of propylene to butyric acid, i. e.,

$$CH_3-CH{=}CH_2 + HCOOH \xrightleftharpoons{\text{catalyst}} CH_3CH_2CH_2COOH + CH_3-\underset{\displaystyle COOH}{\underset{|}{CH}}-CH_3$$

As shown above, a mixture of primary and secondary carboxylic acids is obtained. Typically, primary acids are formed over secondary acids in a ratio of about 2 : 1. Commercially available formic acid is suitable. Water content, however, should be less than about 10 wt.% since excess water may lead to decreased yields.

The present process may be conducted at temperatures of from 150 to 300° C, preferably from 170 to 230° C and especially from 150 to 300° C, preferably from 170 to 230° C and especially from 190 to 210° C. Lower temperatures typically require longer reaction times and higher temperatures may result in catalyst instability. Carbon, monoxide, nitrogen, Group O noble gases, carbon dioxide, hydrogen, $C_1-C_6$ hydrocarbons and the like may optionally be present as inert gases. Oxygen should be maintained at low levels ($<$ 50 ppm) if present.

The pressure is autogenous, i. e., that generated by the reactants and any inert gases present at the desired reaction temperature. Such pressures are typically in the range of from 0.1 to 20 MPa (1 MPa = 10 atm). The pressure is not critical and greater pressures can be generated by added gas. There is generally, however, no advantage to operating at high pressures.

Reaction times may vary from 0.1 to 24 hours or more depending on the reaction parameters chosen. Reaction times of from 1 to 10 hours are suitable when operating in preferred temperature ranges.

The process may be operated in either a batchwise or continuous mode with continuous operation being preferred. If carboxylic acid is the desired product, it is preferred to use product carboxylic acid as solvent. This is advantageous since it permits recycle of a portion of the product stream without any expensive separations step. If esters are the desired product, they may be readily separated by conventional techniques such as distillation.

By practising the process of the invention, formate esters can be converted to longer chain esters by reaction with olefin. Similarly, formic acid can be converted to a longer chain carboxylic acid, which can then be further reacted to form an ester, if desired. Neither reaction requires the presence of CO or hydroxyl containing compound. Moreover, no special high pressure equipment is needed. These processes are further exemplified by the following examples.

## Example 1

A one-liter, Hastelloy-C, stirred autoclave was charged with the following reactants, solvent and catalyst:

| | |
|---|---|
| 0.5 gm. | cyclooctadiene iridium (I) chloride |
| 75 gms. | 95—97% formic acid |
| 10 ml. | methyl iodide |
| 300 g. | glacial acetic acid |

The autoclave was flushed with nitrogen, sealed, charged with ethylene until the pressure reached 3 MPa at room temperature, and heated with stirring, to 200°C. At this temperature the pressure developed was 6.0 MPa (1 MPa = 10 atm.). During the next 1.5 hours the pressure dropped to 2.4 MPa. After four hours, further pressure drop was slight and the heating was discontinued. Upon cooling to ambient temperature and venting excess gases, the liquid contents were removed.

The product weighed 425 gms. Analysis by gas liquid chromatography (thermal conductivity dector, Poropak T column), with dimethyl formamide as internal standard, indicated the presence of 100.6 gms. of propionic acid (90% yield). No significant quantities of by-products were indicated.

## Example 2

A one-liter, Hastelloy C, stirred autoclave was charged with the following reactants, solvent and catalyst:

0.5 gm. cyclooctadiene iridium (I) chloride
75 gms. 95—97% formic acid
15 ml. methyl iodide
250 gm. propionic acid

The autoclave was flushed with nitrogen and sealed. Propylene (66 gms.) was added as a liquid from another pressure vessel. Stirring was begun and the vessel and contents were heated to 200°C and held at this temperature for four hours. At this temperature, the pressure dropped from an initial 3.3 MPa very quickly to 1.8 MPa, and then rose slowly to a final value of 3.1 MPa.

The vessel wa cooled to ambient temperature, the gases were vented and a sample was taken for analysis by a mass spectrograph. The gas was 67 mole % carbon monoxide, 12 mole % carbon dioxide, 3.9 mole % propylene, 4.3 mole % propane and 1.0 mole % hydrogen. The liquid contents weighed 400 grams. Analysis by gas liquid chromatography (glc) indicated the presence of 51 gms. of n-butyric acid, and 25 gms. of isobutyric acid. Further confirmation of the presence and identity of these acid products were obtained through analysis by gc-mass spectrograph combination. Other compounds present in lesser quantities were isopropyl iodide, isopropyl propionates and isopropyl iso- and n-butyrate.

## Examples 3—15

In each of the following examples, the solution of cyclooctadiene iridium (I) chloride dimer (0.50 g), propionic acid (300 g), 95—97% formic acid and a variable amount of methyl iodide was charged to a one-liter Hastelloy-C, stirred autoclave. The autoclave was flushed with nitrogen, sealed and then charged with butene-1 as a liquid from another pressure vessel.

Stirring was begun and the vessel and contents were heated to reaction temperature and held at that temperature for four hours. Samples were taken after 1/2, one and two hours at temperature. These were each analyzed by glc with a Poropak-T column programmed from 170°C to 195°C and a thermal conductivity detector.

The vessel and contents were cooled to room temperature. Residual gases were vented. The liquid contents were removed and analyzed by glc. The results are given in Table I.

Table I

| Example No. | HCOOH g | $CH_3I$ g | Olefin g | Temp. °C | Time Hr | Iso-acid g | n-acid g |
|---|---|---|---|---|---|---|---|
| 3[a]) | 144 | 18.2 | 64 | 195 | 0.5 | 7.7 | 18.2 |
| | | | | | 1.0 | 9.7 | 22.1 |
| | | | | | 2.0 | 16.7 | 29.5 |
| | | | | | 4.0 | 27.6 | 34.6 |

Table I (Continuation)

| Example No. | HCOOH g | $CH_3I$ g | Olefin g | Temp. °C | Time Hr | Iso-acid g | n-acid g |
|---|---|---|---|---|---|---|---|
| 4 | 72 | 9.1 | 62 | 195 | 0.5 | 11.1 | 29.5 |
| | | | | | 1.0 | 16.2 | 40.9 |
| | | | | | 2.0 | 19.3 | 50.2 |
| | | | | | 4.0 | 21.3 | 53.6 |
| 5 | 72 | 4.5 | 63 | 195 | 0.5 | 16.5 | 38.1 |
| | | | | | 1.0 | 20.8 | 52.6 |
| | | | | | 2.0 | 23.2 | 61.8 |
| | | | | | 4.0 | 24.8 | 67.0 |
| 6[b]) | 72 | 18.1 | 122 | 195 | 0.5 | 22.5 | 63.7 |
| | | | | | 1.0 | 30.4 | 83.8 |
| | | | | | 2.0 | 33.0 | 90.4 |
| | | | | | 4.0 | 31.6 | 83.4 |
| 7 | 72 | 18.1 | 124 | 195 | 0.5 | 26.5 | 47.3 |
| | | | | | 1.0 | 34.9 | 70.9 |
| | | | | | 2.0 | 38.2 | 84.4 |
| | | | | | 4.0 | 38.2 | 85.1 |
| 8 | 72 | 45.4 | 57 | 180 | 0.5 | 3.7 | 8.9 |
| | | | | | 1.0 | 4.4 | 8.9 |
| | | | | | 2.0 | 5.5 | 12.1 |
| 9 | 72 | 4.5 | 66 | 180 | 0.5 | 4.8 | 10.7 |
| | | | | | 1.0 | 9.2 | 21.1 |
| | | | | | 2.0 | 15.8 | 39.2 |
| | | | | | 5.0 | 22.9 | 56.3 |
| 10 | 72 | 18.1 | 53 | 180 | 0.5 | 6.4 | 13.2 |
| | | | | | 1.0 | 8.0 | 18.2 |
| | | | | | 2.0 | 10.6 | 21.7 |
| | | | | | 4.0 | 11.6 | 25.6 |

Table I (Continuation)

| Example No. | HCOOH g | $CH_3I$ g | Olefin g | Temp. °C | Time Hr | Iso-acid g | n-acid g |
|---|---|---|---|---|---|---|---|
| 11 | 72 | 2.3 | 66 | 195 | 0.5 | 15.7 | 39.3 |
| | | | | | 1.0 | 20.6 | 53.4 |
| | | | | | 2.0 | 25.1 | 66.5 |
| | | | | | 4.0 | 27.0 | 75.5 |
| 12 | 72 | 1.0 | 66 | 195 | 0.5 | 10.4 | 25.1 |
| | | | | | 1.0 | 21.2 | 52.0 |
| | | | | | 2.0 | 32.7 | 73.3 |
| | | | | | 4.0 | 34.5 | 77.5 |
| 13 | 72 | 0.5 | 66 | 195 | 0.5 | 6.7 | 26.6 |
| | | | | | 1.0 | 13.6 | 48.6 |
| | | | | | 2.0 | 21.7 | 70.3 |
| | | | | | 4.0 | 24.2 | 82.2 |
| 14 | 72 | 0.4 | 72 | 195 | 0.5 | 2.7 | 13.4 |
| | | | | | 1.0 | 7.8 | 36.7 |
| | | | | | 2.0 | 20.7 | 73.8 |
| | | | | | 4.0 | 25.7 | 89.4 |
| 15[c]) | 43 | 0 | 43 | 195 | 0.5 | 0 | 0 |

[a]) Solvent: 225 g propionic acid in this example.
[b]) Propionic anhydride, 25 g, was added to dehydrate the commercial 95—97% formic acid.
[c]) This reaction was conducted with the use of a glass liner in the autoclave. The catalyst was 0.8 g $(Ph_3P)_2(Ir(CO)I$ used as purchased from Strem Chemical Co. The iodide is equivalent to 0.13 g $CH_3I$ or 1 iodide per iridium atom. The solvent was 180 g of propionic acid.

Examples 4, 5, 8, 9, 11, 12 and 13 illustrate the effect of varying the amount of methyl iodide. Examples 8 and 10 show that very high concentrations of methyl iodide (100—200 I/Ir) retard both rate and ultimate yield. Examples 4 and 5 show that I/Ir ranges of 20—40 give good initial rates but not optimal yields. Examples 11, 12, 13 and 14 demonstrate that an I/Ir range of 2—10 gives the best results. Example 15 demonstrates the inferiority of an I/Ir ratio of unity.

Examples 8,9 and 10 show that the reaction operates well at a lower temperature.

## Example 16

The procedure of examples 4—15 was followed, except that the reacting charge consisted of

| | |
|---|---|
| $[Ir(COD)Cl]_2$ | 0.50 g |
| 95—97% formic acid | 72 g |
| butene-2 | 64 g |
| propionic acid | 300 g |
| methyl iodide | 4.5 g |

The reaction was conducted at 195°C for four hours, with sampling and glc analyses after $^1/_2$, one and two hours. The final sample was also analyzed after cooling and removed from the autoclave. The results obtained are given in Table II. A comparison of these results with those of Exemple 5.

Table II

| Time (hrs) | g iso-acid | g n-acid |
|---|---|---|
| 0.5 | 14.2 | 32.1 |
| 1.0 | 19.3 | 55.7 |
| 2.0 | 24.9 | 58.5 |
| 4.0 | 25.2 | 68.8 |

Table I shows that the productivities and isomer distributions are nearly identical. Thus it is proved that internal olefins are operable in the process.

## Example 17

The process of Example 5 was repeated, except that the iridium catalyst utilized was hexachloroiridic acid, $H_2IrCl_6$, 0.5 g (1.2 mmol), and the solvent was 300 g of acetic acid. The amount of butene-1 was 67 g. The product distribution is shown in Table III. These data prove that different complexes of soluble iridium are operable in the process. It is also shown that acetic acid is a satisfactory solvent.

Table III

| Time (hrs) | g iso-acid | g n-acid |
|---|---|---|
| 0.5 | 9.5 | 25.2 |
| 1.0 | 14.2 | 40.1 |
| 2.0 | 17.3 | 47.6 |
| 4.0 | 18.3 | 50.2 |

## Example 18

The process of Example 5 was repeated, exept that the solvent was 300 g of valeric acid, $CH_3CH_2CH_2CH_2COOH$, and the olefin was propylene (64 g). After the four-hour reaction period, the final solution contained 40.6 g of isobutyric acid and 84.8 g of butyric acid (94% total yield). This example shows that valeric acid is suitable as a solvent and that propylene is a suitable olefin.

Example 19

In this example, the catalyst, reactants and solvent charged to the autoclave were 65 g of propylene, 72 g of 95—97% formic acid, 0.50 g of bis(triphenylphosphine)iridium(I)carbonyl chloride and 150 g of propionic acid. A glass liner was utilized in the autoclave. After only one-half hour at this temperature the pressure had risen to 13.55 MPa. The reaction was stopped by cooling the reactor and contens to room temperature. The product (125 g) consisted of a colorless liquid and black solid. This example proves the necessity of iodide promoter.

Example 20

A solution of 120 g of methyl formate, 36 g of 95% formatic acid, 0.50 g of cyclooctadiene iridium (I) chloride dimer, 4.5 g of methyl iodide, 58 g of propylene in 250 g of propionic acid was charged to the one-liter Hastelloy-C, stirred autoclave. After flushing with nitrogen, stirring was applied and the autoclave and contents were heated to 195°C. Samples were taken after $^1/_2$, one and two hours of reaction time. After four hours the pressure had dropped from 3.7 MPa to 2.2 MPa. The vessel and contents were cooled to ambient temperature and the excess gases were vendet. The product solution was removed (wt. 456 g). The product and the samples were analyzed by glc. The results are summarized in Table IV.

Table IV

| Time (hrs) | g methyl isobutyrate | g methyl butyrate | g isobutyric acid | g butyric acid | g acetic acid |
|---|---|---|---|---|---|
| 0.5 | 0.2 | 1.5 | 3.6 | 7.8 | 3.4 |
| 1.0 | 0.7 | 3.2 | 7.3 | 16.0 | 6.1 |
| 2.0 | 1.8 | 8.3 | 15.4 | 34.3 | 19.2 |
| 4.0 | 3.1 | 10.5 | 20.6 | 47.4 | 65.5 |

These results prove that the isomerization of methyl formate to acetic acid and the addition of methyl formate and formic acid to olefins can be effected in a single operation and that methyl formate as well as formic acid can be added to propylene.

Example 21

The procedure of Example 5 was followed except that the olefin was octene-1 (112 g) and the reaction temperature was 200° C. Analysis of the final solution indicated the presence of 70 g of linear nonanoic acid and 47 g of branched-chain nonanoic acids. This experiment demonstrates the applicability of higher carbonchain olefins.

Example 22

A solution was prepared containing 250 ml. of acetic acid as solvent, 100 ml. of methyl formate, 15 ml. of methyl iodide and 0.30 gms. of bis [cyclooctadiene iridium (I) cloride]. This solution was placed in a one-liter, Hastelloy-C-lined autoclave and the atmosphere flushed with nitrogen. It was heated to 200°C at which temperature the pressure was 2.0 MPa. Ethylene was added to a total pressure of 5.2 MPa. The heating was continued for eight hours and the pressure was maintained between 4.6—5.2 MPa by periodic addition of ethylene.

The vessel and contents were cooled to room temperature and the excess gas vented. The residual amber solution weighed 405 g and a gc analysis showed that about 10% of the contens was methyl propionate.

## Claims

1. A homogeneous process for preparing a carboxylic acid ester which comprises contacting an olefin and a formate ester with a catalytically effective amount of a soluble iridium salt or compound and an iodide promoter in the presence of a carboxylic acid at a temperature of from 150 to 300°C.

2. A process according to claim 1 wherein the formate ester has the formula $HCOOR^1$ where $R^1$ is $C_1-C_{20}$ aliphatic, $C_3-C_{12}$ cycloaliphatic, $C_7-C_{16}$ araliphatic or $C_6-C_{12}$ aryl.

3. A homogeneous process for preparing a carboxylic acid which comprise contacting an olefin and formic acid with a catalytically effective amount of a soluble iridium salt or compound and an iodide promoter at a temperatur of from 150 to 300°C.

4. A homogeneous process for preparing a carboxylic acid which comprises contacting an olefin and formic acid with a catalytically effective amount of a soluble iridium salt or compound and an iodide promoter at a temperatur of from 150 to 300°C in the presence of a solvent comprising a carboxylic acid having at least two carbon atoms per molecule.

5. A process according to claim 4 wherein the carboxylic acid used as a solvent has the formula RCOOH where R is $C_1-C_{20}$ aliphatic.

6. A process according to any one of the preceding claims wherein the olefins has the formula $R^2R^3C = CR^4R^5$ where $R^2$, $R^3$, $R^4$ and $R^5$ are independently hydrogen, $C_1-C_{12}$ aliphatic, $C_3-C_{10}$ cycloaliphatic, $C_7-C_{14}$ araliphatic or $C_6-C_{10}$ aryl with the proviso that at least two of $R^2$ to $R^5$ are hydrogen and with the further proviso that any two of $R^2$ to $R^5$ may be joined together to form a $C_3$ to $C_{10}$ ring.

7. A process according to any one of the preceding claims wherein the iodide promoter is iodide, HI, organic iodide, ammonium iodide or phosphonium iodide.

8. A process according to any one of the preceding claims wherein the iridium salt or compound is a complex containing halide, phosphine, amine, arsine, cyanide, sulfide, ester, betadiketonate, carboxylate, hydride, sulfite, nitro, carbonyl, alkyl, alkene or alkyne as ligands.

9. A process according to claim 8 wherein the salt or compound is $[Ir(cyclooctadiene)Cl]_2$, $IrCl_3 \cdot x H_2O$ $IrBr_3 \cdot x H_2O$ or $IrI_3 \cdot x H_2O$ where x is from 0 to 12.

## Patentansprüche

1. Homogenes Verfahren zur Herstellung eines Carbonsäureesters, dadurch gekennzeichnet, daß ein Olefin und ein Formiatester mit einer katalytisch wirksamen Menge eines löslichen Iridiumsalzes oder einer löslichen Iridiumverbindung und einem Jodidpromotor in Gegenwart einer Carbonsäure bei einer Temperatur von 150 bis 300°C kontaktiert werden.

2. Verfahren nach Anspruch 1, dadurch gekennzeichnet, daß der Formiatester die Formel $HCOOR^1$ besitzt, in der $R^1$ eine aliphatische $C_1-C_{20}$-Gruppe, eine cycloaliphatische $C_3-C_{12}$-Gruppe, eine araliphatische $C_7-C_{16}$-Gruppe oder eine $C_6-C_{12}$-Arylgruppe ist.

3. Homogenes Verfahren zur Herstellung einer Carbonsäure, dadurch gekennzeichnet, daß ein Olefin und Ameisensäure mit einer katalytisch wirksamen Menge eines löslichen Iridiumsalzes oder einer löslichen Iridiumverbindung und einem Jodidpromotor bei einer Temperatur von 150 bis 300°C kontaktiert werden.

4. Homogenes Verfahren zur Herstellung einer Carbonsäure, dadurch gekennzeichnet, daß ein Olefin und Ameisensäure mit einer katalytisch wirksamen Menge eines löslichen Iridiumsalzes oder einer löslichen Iridiumverbindung und einem Jodidpromotor bei einer Temperatur von 150 bis 300°C in Gegenwart eines Lösungsmittels kontaktiert werden, das eine Carbonsäure mit mindestens 2 Kohlenstoffatomen je Molekül umfaßt.

5. Verfahren nach Anspruch 4, dadurch gekennzeichnet, daß die als Lösungsmittel verwendete Carbonsäure die Formel RCOOH besitzt, in der R eine aliphatische $C_1-C_{20}$-Gruppe ist.

6. Verfahren nach einem der vorangehenden Ansprüche, dadurch gekennzeichnet, daß das Olefin die Formel $R^2R^3C = CR^4R^5$ besitzt, in der $R^2$, $R^3$, $R^4$ und $R^5$ unabhängig voneinander Wasserstoff, eine aliphatische $C_1-C_{12}$-Gruppe, eine cycloaliphatische $C_3-C_{10}$-Gruppe, eine araliphatische $C_7-C_{14}$-Gruppe oder eine $C_6-C_{10}$-Arylgruppe mit der Maßgabe sind, daß mindestens zwei von $R^2$ bis $R^5$ Wasserstoff sind, und mit der weiteren Maßgabe, daß jeweils zwei von $R^2$ bis $R^5$ unter Bildung eines $C_3-C_{10}$-Rings miteinander verbunden sein können.

7. Verfahren nach einem der vorangehenden Ansprüche, dadurch gekennzeichnet, daß der Jodidpromotor Jod, HI, organisches Jodid, Ammoniumjodid oder Phosphoniumjodid ist.

8. Verfahren nach einem der vorangehenden Ansprüche, dadurch gekennzeichnet, daß das Iridiumsalz oder die Iridiumverbindung ein Komplex ist, der Halogenid, Phosphin, Amin, Arsin, Cyanid, Sulfid, Ester, Betadiketonat, Carboxylat, Hydrid, Sulfit, Nitrogruppen, Carbonylgruppen, Alkylgruppen, Alken oder Alkin als Liganden enthält.

9. Verfahren nach Anspruch 8, dadurch gekennzeichnet, daß das Salz oder die Verbindung $[Ir(cyclooctadien)Cl]_2$, $IrCl_3 \cdot x H_2O$, $IrBr_3 \cdot x H_2O$ oder $IrI_3 \cdot x H_2O$ ist, wobei x 0 bis 12 ist.

**Revendications**

1. Procédé homogène pour préparer un ester d'acide carboxylique, qui consiste à mettre en contact une oléfine et un ester formiate avec une quantité catalytiquement efficace d'un sel ou d'un composé soluble de l'iridium, et d'un promoteur iodure, en présence d'un acide carboxylique à une température de 150 à 300° C.

2. Procédé selon la revendication 1, dans lequel l'ester formiate a la formule $HCOOR^1$, où $R^1$ est un radical aliphatique en $C_1-C_{20}$, cycloaliphatique en $C_3-C_{12}$, araliphatique en $C_7-C_{16}$ ou aryle en $C_6-C_{12}$.

3. Procédé homogène pour la préparation d'un acide carboxylique, qui consiste à mettre en contact une oléfine et de l'acide formique avec une quantité catalytiquement efficace d'un sel ou composé soluble de l'iridium et un promoteur iodure à une température de 150 à 300° C.

4. Procédé homogène pour la préparation d'un acide carboxylique, qui consiste à mettre en contact une oléfine et de l'acide formique avec une quantité catalytiquement efficace d'un sel ou composé soluble de l'iridium et d'un promoteur iodure à une température de 150 à 300° C, en présence d'un solvant comprenant un acide carboxylique ayant au moins deux atomes de carbone par molécule.

5. Procédé selon la revendication 4, dans lequel l'acide carboxylique utilisé comme solvant a la formule RCOOH, où R est un radical aliphatique en $C_1-C_{20}$.

6. Procédé selon l'une quelconque des revendications précédentes dans lequel l'oléfine a la formule $R^2R^3C = CR^4R^5$, où $R^2$, $R^3$, $R^4$ et $R^5$ sont, indépendamment les uns des autres, l'hydrogène, un radical aliphatique en $C_1-C_{12}$, cycloaliphatique en $C_3-C_{10}$, araliphatique en $C_7-C_{14}$ ou aryle en $C_6-C_{10}$, à la condition qu'au moins deux des radicaux $R^2$ à $R^5$ soient l'hydrogène, et à la condition supplémentaire que deux quelconques des radicaux $R^2$ à $R^5$ puissent être réunis l'une à l'autre pour former un cycle en $C_3$ à $C_{10}$.

7. Procédé selon l'une quelconque des revendications précédentes, dans lequel le promoteur iodure est l'iode, HI, un iodure organique, l'iodure d'ammonium ou l'iodure de phosphonium.

8. Procédé selon l'une quelconque des revendications précédentes, dans lequel le sel ou composé de l'iridium est un complexe comprenant, en tant que ligands, des halogénures, phosphines, amines, arsines, cyanures, sulfures, esters, bêta-dicétonates, carboxylates, hydrures, sulfites, nitro, ou les radicaux carbonyle, alkyle, alcène ou alcyne.

9. Procédé selon la revendication 8, dans lequel le sel ou composé est $[Ir(cyclooctadiène)Cl]_2$, $IrCl_3 \cdot x H_2O$, $IrBr_3 \cdot x H_2O$ ou $IrI_3 \cdot x H_2O$, où x vaut de 0 à 12.